# EUROPEAN PATENT APPLICATION

(11) **EP 2 698 103 A1**
(43) Date of publication of application: **19.02.2014**
(21) Application number: 13180332.2
(22) Date of filing: 14.08.2013
(51) Int. Cl.: A61B 5/00, A61B 5/055, A61B 6/00, A61B 8/08

(54) **System and method for MRI imaging using polarized light**

(30) Priority: 15.08.2012 IL 22149312
(71) Applicant: Aspect Imaging Ltd., 60850 Shoham (IL)
(72) Inventor: Rapoport, Uri, 73115 Moshav Ben Shemen (IL); Batt, Aryeh, 90440 Har Hevron (IL)
(74) Representative: Lecomte & Partners

(57) **Abstract**

The present invention provides an MRI imaging system, comprising an MRI device adapted to image at least a portion of an animal; a photon source; an imaging photon detector, said photon detector capable of detecting photons emitted by said photon source; and an image processor adapted to superimpose said MRI image and said photon detector image, said superimposed image with given SNR₀; wherein said system further comprises one or more polarizers located between said animal and said photon detector, and wherein a value of SNR₁ for said superimposed image is obtained, SNR₁ being greater than SNR₀.

## Description

### FIELD OF THE INVENTION

The present invention generally pertains to a system and method for MRI imaging the body of an animal using polarized light to provide images with high signal to noise ratio and high contrast.

### BACKGROUND OF THE INVENTION

In conventional MRI systems, systems with high magnetic field (approximately 13T) provide images with high signal to noise ratio (SNR) but low contrast. Systems with low magnetic field (approximately 1T) provide images with much lower SNR but higher contrast. Both low SNR and low contrast can make it difficult to understand MRI images.

There is a further disadvantage in that systems with high magnetic field can damage animal, so that very high magnetic fields are better avoided.

Patent application US 20110073764A1 discloses a method and system for nuclear imaging normally involve detection of energy by producing at most two or three bursts of photons at a time in response to events including incident gamma radiation. F number of sharing central groups of seven photodetectors, depending on the photodetector array size, is arranged in a honeycomb array for viewing zones of up to F bursts of optical photons at a time for each continuous detector and converting the bursts of optical photons into signal outputs, where each of the central groups is associated with a zone. This enables the detector sensitivity to be increased by as much as two orders of magnitude, and to exchange some of this excess sensitivity to achieve spatial resolution comparable to those in CT and MRI, which would be unprecedented. Signal outputs that are due to scattered incident radiation are rejected for each of the central groups to reduce image blurring, thereby further improving image quality. For planar imaging, the energy and position signals of up to the F number of valid events are generated once every deadtime period and transferred to computer memory for image display and data analysis. The number of valid events detected is up to 6F for SPECT and up to 3F for PET imaging. However, it does not disclose combining MRI with both direct imaging and fluorescence imaging.

Patent application US 20110021970A1 discloses products, compositions, systems, and methods for modifying a target structure which mediates or is associated with a biological activity, including treatment of conditions, disorders, or diseases mediated by or associated with a target structure, such as a virus, cell, subcellular structure or extracellular structure. The methods may be performed in situ in a non-invasive manner by placing a nanoparticle having a metallic shell on at least a fraction of a surface in a vicinity of a target structure in a subject and applying an initiation energy to a subject thus producing an effect on or change to the target structure directly or via a modulation agent. The nanoparticle is configured, upon exposure to a first wavelength X1; to generate a second wavelength X2 of radiation having a higher energy than the first wavelength\l. The methods may further be performed by application of initiation energy to a subject in situ to activate a pharmaceutical agent directly or via an energy modulation agent, optionally in the presence of one or more plasmonics active agents, thus producing an effect on or change to the target structure. Kits containing products or compositions formulated or configured and systems for use in practicing these methods. However, it does not disclose combining MRI with both direct imaging and fluorescence imaging.

It is therefore a long felt need to provide a system which does not suffer from low SNR and also does not suffer from low contrast.

### SUMMARY OF THE INVENTION

It is an object of the present invention to disclose a system and method for MRI imaging the body of an animal using polarized light to provide images with high signal to noise ratio and high contrast.

It is an object of the present invention to disclose an MRI imaging system, comprising an MRI device adapted to image at least a portion of an animal; a photon source; an imaging photon detector, said photon detector capable of detecting photons emitted by said photon source; and an image processor adapted to superimpose said MRI image and said photon detector image, said superimposed image with given SNR₀; wherein said system further comprises one or more polarizers located between said animal and said photon detector, and wherein a value of SNR₁ for said superimposed image is obtained, SNR₁ being greater than SNR₀.

It is an object of the present invention to disclose the MRI imaging system, additionally comprising a second imaging photon detector capable of detecting fluorescent photons.

It is an object of the present invention to disclose the MRI imaging system, additionally comprising a second polarizer located between said animal and said second imaging photon detector.

It is an object of the present invention to disclose the MRI imaging system, wherein photons emitted by said photon source and said fluorescent photons are detected by a single photon detector.

It is an object of the present invention to disclose the MRI imaging system, wherein a third polarizer is located between said photon source and said subject.

It is an object of the present invention to disclose the MRI imaging system, wherein said MRI magnets are selected from the group consisting of superconducting magnets, permanent magnets, and any combination thereof.

It is an object of the present invention to disclose the MRI imaging system, wherein said polarizer is selected from the group consisting of birefringent crystals, polarizing crystals, polarizers, and any combination thereof.

It is an object of the present invention to disclose the MRI imaging system, wherein the wavelength range of said photon source is selected from the group consisting of gamma radiation, X-radiation, far UV, near UV, visible light, near IR, far IR, and any combination thereof.

It is an object of the present invention to disclose the MRI imaging system, wherein said photon detector is selected from the group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose the MRI imaging system, wherein said image processor is adapted to render said superimposed image by a Boolean method of correlating or combining at least a portion of said MRI image and at least a portion of said photon detector image.

It is an object of the present invention to disclose the MRI imaging system, wherein said image processor is adapted to render said superimposed image by a Boolean method of correlating or combining at least a portion of said MRI image and at least a portion of at least one of the group consisting of said photon detector image and said second photon detector image.

It is an object of the present invention to disclose the MRI imaging system, wherein said Boolean method uses Boolean operators selected from the group consisting of OR, AND, NOT, EXCLUSIVE OR and any combination thereof.

It is an object of the present invention to disclose a method for MRI imaging at least a portion of an animal, said method is characterized by steps of providing an MRI imaging system with an MRI device adapted to image at least a portion of said animal; a photon source; an imaging photon detector, said photon detector capable of detecting photons emitted by said photon source; and an image processor adapted to superimpose said MRI image and said photon detector image, said superimposed image with given SNR₀; locating one or more polarizers between said animal and said photon detector, acquiring an MRI image of said at least a portion of said animal; acquiring a photon detector image of said at least a portion of said animal; and superimposing said MRI image and said photon detector image of said at least a portion of said animal, thereby obtaining a value of SNR₁ for said superimposed image; SNR1 being greater than SNR₀.

It is an object of the present invention to disclose the method for MRI imaging, comprising an additional step of providing a second imaging photon detector, capable of detecting fluorescent photons.

It is an object of the present invention to disclose the method for MRI imaging, comprising an additional step of providing a second polarizer between said animal and said second imaging photon detector.

It is an object of the present invention to disclose the method for MRI imaging, comprising an additional step of detecting said photons emitted by said photon source and said fluorescent photons with a single imaging detector.

It is an object of the present invention to disclose the method for MRI imaging, comprising an additional step of injecting said subject with fluorescence-inducing material.

It is an object of the present invention to disclose the method for MRI imaging, comprising an additional step of providing a third polarizer between said photon source and said subject. It is an object of the present invention to disclose the method for MRI imaging, comprising an additional step of selecting said MRI magnets from the group consisting of superconducting magnets, permanent magnets, and any combination thereof.

It is an object of the present invention to disclose the method for MRI imaging, comprising an additional step of selecting said polarizer from the group consisting of birefringent crystals, polarizing crystals, polarizers, and any combination thereof.

It is an object of the present invention to disclose the method for MRI imaging, comprising an additional step of selecting the wavelength range of said photon source from the group consisting of gamma radiation, X-radiation, far UV, near UV, visible light, near IR, far IR, and any combination thereof.

It is an object of the present invention to disclose the method for MRI imaging, comprising an additional step of selecting said at least one imaging detector from the group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose the method for MRI imaging, additionally comprising a step of rendering said superimposed image by a Boolean method of correlating or combining at least a portion of said MRI image and at least a portion of said photon detector image.

It is an object of the present invention to disclose the method for MRI imaging, additionally comprising a step of rendering said superimposed image by a Boolean method of correlating or combining at least a portion of said MRI image and at least a portion of at least one of the group consisting of said photon detector image and said second photon detector image.

It is an object of the present invention to disclose the method for MRI imaging, comprising an additional step of using for said Boolean method Boolean operators selected from the group consisting of OR, AND, NOT, EXCLUSIVE OR and any combination thereof.

### BRIEF DESCRIPTION OF THE FIGURES

In order to better understand the invention and its implementation in practice, a plurality of embodiments will now be described, by way of non-limiting example only, with reference to the accompanying drawings, wherein
Fig. 1 schematically illustrates a system of the present invention; and
Fig. 2 depicts a flow diagram of an embodiment of the present invention.

### DETAILED DESCRIPTION OF THE PREFERRED EMBODIEMNTS

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide a means and method for providing functional MRI with high signal to noise ratio and high contrast.

In conventional MRI systems, systems with high magnetic field (from 3 Tesla, e.g., approximately 13T) provide images with high SNR but low contrast. Systems with low magnetic field (less than 3 Tesla, e.g., approximately 1T) provide images with much lower SNR but higher contrast. Both low SNR and low contrast can make it difficult to understand MRI images.

The system of the present invention combines a low-field (1 T) MRI image with camera images, acquired simultaneously with the MRI image. In one embodiment of the present invention, an MRI image is acquired of an animal or a portion of an animal. Simultaneously, the subject or the portion of the subject is irradiated with electromagnetic radiation in at least one wavelength range. Preferred ranges are visible light, near and far UV and near and far IR, although other embodiments can use X-radiation and gamma radiation. The electromagnetic radiation passing through or reflected from the subject or the portion of the subject, or both, passes through a polarizer and is detected by a camera or other suitable image-forming detector. The polarizer can be a Polaroid ™ or other film-type filter, a birefringent crystal, another type of polarizing crystal, or any other suitable polarizing device, as is well known in the art. In the best embodiment of the system of the present invention, the electromagnetic radiation from the source excites fluorophores or other fluorescing molecules within the subject's body. The fluorophores or other fluorescing molecules can occur naturally within the subject's body or can be introduced into the body by any of the methods well known in the art. Fluorescent light which exits the body then passes through a polarizer, as described above, and is detected and imaged. In the best embodiment, a single imaging detector detects both the radiation in the irradiating wavelength range and the fluorescent radiation. In other embodiments, separate detectors are used for the two wavelength ranges.

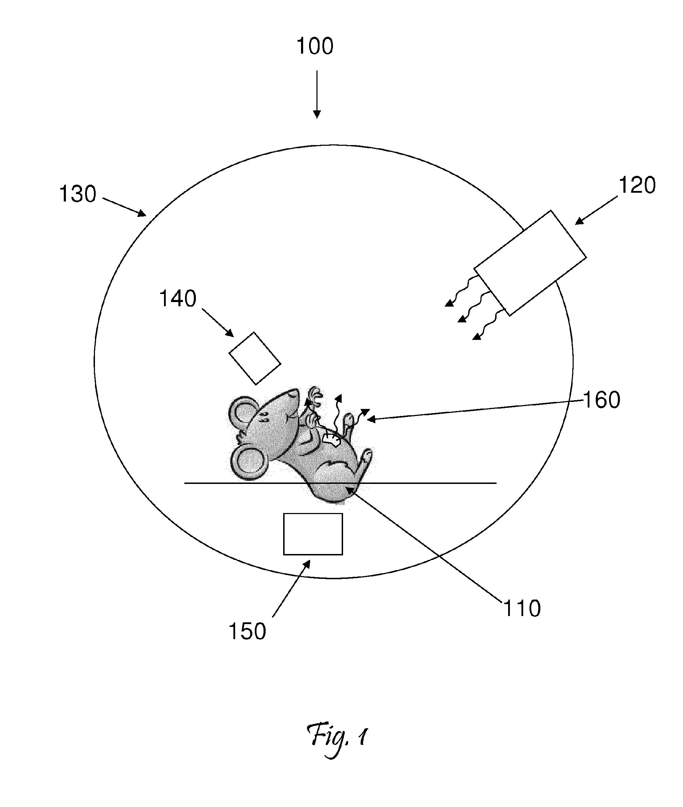

In reference to **Fig. 1**, an embodiment of the device is shown wherein an animal (110) is shown inside an MRI (100) with RF coil 130. The subject is illuminated with light from source 120. Some of this light passes through the subject and is detected by a detector (150). Some of the light is absorbed by fluorophores in the subject and is reradiated as fluorescent light, or is fluorescence otherwise emitted by the subject. The fluorescent radiation is detected by detector 140.

The MRI image, the polarized irradiating-wavelength image and the polarized fluorescent image are then fused, using methods well-known in the art, to provide a combined image with high contrast, high SNR and high resolution.

In one embodiment, the imaging detector is a CCD array. In another embodiment, it is a camera.

In yet other embodiments, multiple imaging detectors are used, each imaging the volume of interest from a different angle.

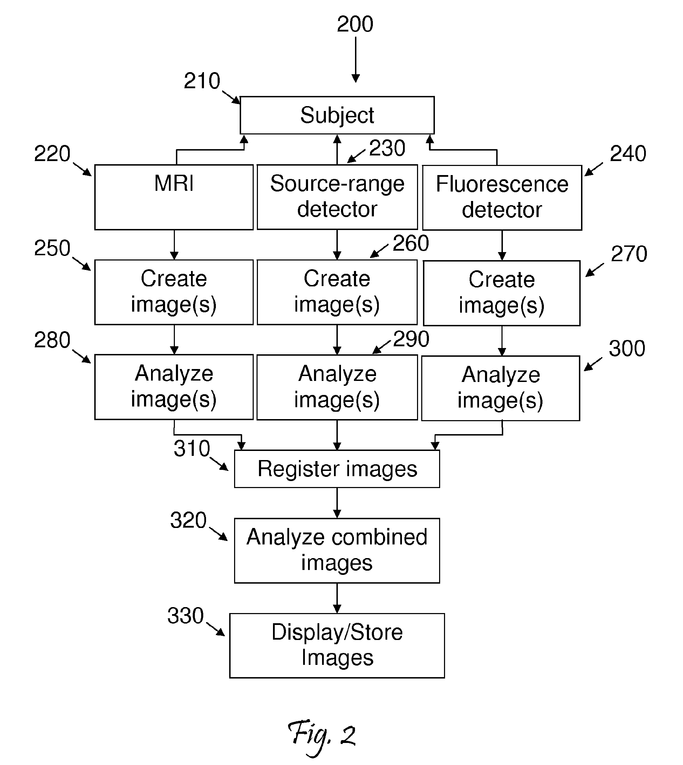

In reference to **Fig. 2****,** a block diagram (200) is shown which outlines a method of operation of the system. Signals are acquired from the subject (210) via MRI (220), via a detector capable of detecting radiation in the wavelength range of a source (230), and via a detector capable of detecting fluorescence from fluorescing material within the subject (240).

The images are created from the signals (250, 260, 270), using techniques well-known in the art. The images are then registered, fused (280) and analyzed (290) to form a composite image, using techniques known in the art, which has the good contrast typical of low-field (1T) MRI images, but a higher resolution and higher SNR than is feasible with low field (1T) MRI alone. The composite image(s) can be displayed and stored (300) for later use.

Fusing techniques include rendering the images using Boolean methods of correlating and combining the images. Combining binary images using Boolean logic makes it possible to select structures or objects based on multiple criteria, such as, but not limited to, masking and threshholding. The Boolean operators commonly used are OR, AND, NOT, EXCLUSIVE OR and combinations thereof.

In some embodiments, the direct-illumination images, fluorescent images, or both are acquired in times on the order of a few tenths of a second to a few tens of seconds, enabling the system of the present invention to observe functional changes in the subject body such as ion transport mechanisms, nerve activity and blood flow.

## Claims

1. An MRI imaging system, comprising an MRI device adapted to image at least a portion of an animal; a photon source; an imaging photon detector, said photon detector capable of detecting photons emitted by said photon source; and an image processor adapted to superimpose said MRI image and said photon detector image, said superimposed image with given SNR₀; wherein said system further comprises one or more polarizers located between said animal and said photon detector, and wherein a value of SNR₁ for said superimposed image is obtained, SNR₁ being greater than SNR₀.

2. The MRI imaging system of claim 1, additionally comprising a second imaging photon detector capable of detecting fluorescent photons.

3. The MRI imaging system of claim 2, additionally comprising a second polarizer located between said animal and said second imaging photon detector.

4. The MRI imaging system of claim 2, wherein photons emitted by said photon source and said fluorescent photons are detected by a single photon detector.

5. The MRI imaging system of claim 3, wherein photons emitted by said photon source and said fluorescent photons are detected by a single photon detector.

6. The MRI imaging system of claim 1, wherein at least one of the following is being held true (a) a third polarizer is located between said photon source and said subject; (b) said MRI magnets are selected from the group consisting of superconducting magnets, permanent magnets, and any combination thereof; (c) said polarizer is selected from the group consisting of birefringent crystals, polarizing crystals, polarizers, and any combination thereof; (d) the wavelength range of said photon source is selected from the group consisting of gamma radiation, X-radiation, far UV, near UV, visible light, near IR, far IR, and any combination thereof; and any combination thereof.

7. The MRI imaging system of claim 1, wherein at least one of the following is being held true (a) said photon detector is selected from the group consisting of a CCD array, a camera, a photoconductive detector array, a photovoltaic detector array, a quantum dot array, a superconducting single-photon detector array, a photovoltaic cell array, a phototube array, and any combination thereof; (b) said image processor is adapted to render said superimposed image by a Boolean method of correlating or combining at least a portion of said MRI image and at least a portion of said photon detector image; and any combination thereof.

8. The MRI imaging system of claim 2, wherein said image processor is adapted to render said superimposed image by a Boolean method of correlating or combining at least a portion of said MRI image and at least a portion of at least one of the group consisting of said photon detector image and said second photon detector image.

9. The MRI imaging system of claim 7, wherein said Boolean method uses Boolean operators selected from the group consisting of OR, AND, NOT, EXCLUSIVE OR and any combination thereof.

10. The MRI imaging system of claim 9, wherein said Boolean method uses Boolean operators selected from the group consisting of OR, AND, NOT, EXCLUSIVE OR and any combination thereof.

11. A method for MRI imaging at least a portion of an animal, said method is **characterized by** steps of:
a. providing an MRI imaging system with an MRI device adapted to image at least a portion of said animal; a photon source; an imaging photon detector, said photon detector capable of detecting photons emitted by said photon source; and an image processor adapted to superimpose said MRI image and said photon detector image, said superimposed image with given SNR₀;
b. locating one or more polarizers between said animal and said photon detector,
c. acquiring an MRI image of said at least a portion of said animal;
d. acquiring a photon detector image of said at least a portion of said animal; and
e. superimposing said MRI image and said photon detector image of said at least a portion of said animal, thereby obtaining a value of SNR₁ for said superimposed image; SNR₁ being greater than SNR₀.

12. The method for MRI imaging of claim 11, comprising an additional step of providing a second imaging photon detector, capable of detecting fluorescent photons.

13. The method for MRI imaging of claim 12, comprising an additional step of providing a second polarizer between said animal and said second imaging photon detector.

14. The method for MRI imaging according of claim 12, comprising an additional step of detecting said photons emitted by said photon source and said fluorescent photons with a single imaging detector.

15. The method for MRI imaging according to claim 13, comprising an additional step of detecting said photons emitted by said photon source and said fluorescent photons with a single imaging detector.

16. The method for MRI imaging of claim 11, additionally comprising at least one step selected from a group consisting of (a) injecting said subject with fluorescence-inducing material; (b) providing a third polarizer between said photon source and said subject; (c) selecting said MRI magnets from the group consisting of superconducting magnets, permanent magnets, and any combination thereof; (d) selecting said polarizer from the group consisting of birefringent crystals, polarizing crystals, polarizers, and any combination thereof; (e) selecting the wavelength range of said photon source from the group consisting of gamma radiation, X-radiation, far UV, near UV, visible light, near IR, far IR, and any combination thereof; (f) selecting said at least one imaging detector from the group consisting of a CCD array, a camera, a photoconductive detector array, a photovoltaic detector array, a quantum dot array, a superconducting single-photon detector array, a photovoltaic cell array, a phototube array, and any combination thereof; (g) rendering said superimposed image by a Boolean method of correlating or combining at least a portion of said MRI image and at least a portion of said photon detector image; (h) rendering said superimposed image by a Boolean method of correlating or combining at least a portion of said MRI image and at least a portion of at least one of the group consisting of said photon detector image and said second photon detector image; (i) using for said Boolean method Boolean operators selected from the group consisting of OR, AND, NOT, EXCLUSIVE OR and any combination thereof; and any combination thereof.
